# EUROPEAN PATENT SPECIFICATION

(11) **EP 3 419 671 B1**
(45) Date of publication and mention of the grant of the patent: **17.02.2021**
(21) Application number: 17710832.1
(22) Date of filing: 24.02.2017
(51) Int. Cl.: A61K 47/69, A61P 15/00, A61K 9/46, A61K 9/00, A61K 9/16, A61K 9/20, A61K 47/40

(54) **HIGH BIOAVAILABILITY OROMUCOSAL PHARMACEUTICAL PREPARATIONS BASED ON CYCLODEXTRIN AND SUCRALOSE**
OROMUKOSALE PHARMAZEUTISCHE PRÄPARATE MIT HOHER BIOVERFÜGBARKEIT AUF BASIS VON CYCLODEXTRIN UND SUCRALOSE
PRÉPARATIONS PHARMACEUTIQUES OROMUQUEUSES À BIODISPONIBILITÉ ÉLEVÉE À BASE DE CYCLODEXTRINE ET DE SUCRALOSE

(30) Priority: 24.02.2016 IT UB20161027
(43) Date of publication of application: 02.01.2019
(73) Proprietor: Altergon S.A., 6901 Lugano (CH)
(72) Inventor: BELLORINI, Lorenzo, 21025 Comerio (VA) (IT); NOCELLI, Luca, 21016 Luino (VA) (IT); FOSSATI, Tiziano, 22010 Moltrasio (IT)
(74) Representative: Gerli, Paolo
(86) International application number: PCT/EP2017/054274
(87) International publication number: WO 2017/144636

(56) References cited:
- EP-A2- 0 375 122
- WO-A2-2011/056978
- US-A- 4 596 795
- US-A1- 2006 120 967
- US-A1- 2015 297 752
- US-B2- 9 066 858
- PREIS MAREN ET AL: "In-vitroandin-vivoevaluation of taste-masked cetirizine hydrochloride formulated in oral lyophilisates", INTERNATIONAL JOURNAL OF PHARMACEUTICS, ELSEVIER BV, NL, vol. 491, no. 1, 5 June 2015 (2015-06-05), pages 8-16, XP029248654, ISSN: 0378-5173, DOI: 10.1016/J.IJPHARM.2015.06.002
- A. MAHESH ET AL: "Development of Taste Masked Fast Disintegrating Films of Levocetirizine Dihydrochloride for Oral Use", CURRENT DRUG DELIVERY, vol. 7, no. 1, 1 January 2010 (2010-01-01) , pages 21-27, XP055313722, NL ISSN: 1567-2018, DOI: 10.2174/156720110790396454
- Patil JS(1), Suresh S.: "Physicochemical Characterization, in vitro Release and Permeation Studies of Respirable Rifampicin-Cyclodextrin Inclusion Complexes", Indian J Pharm Sci. 2009 Nov;71(6):638-43. doi: 10.4103/0250-474X.59545. , 1 September 2009 (2009-09-01), XP055313724, Retrieved from the Internet: URL:https://www.ncbi.nlm.nih.gov/pmc/artic les/PMC2846468/ [retrieved on 2016-10-25]

## Description

### TECHNICAL FIELD

The present invention relates to field of pharmaceutical oromucosal compositions, ingredients and method of production thereof. The invention is based on identification of a new pharmaceutical composite having high bioavailability, and on the use of said composite in the preparation of pharmaceutical oromucosal compositions.

### STATE OF THE ART

Administration of active ingredients should be made taking into account different factors, among which: active ingredients solubility in the biological medium they come into contact with at the time of their administration, active substances permeability through biological membranes in order to reach the systemic circulation, pre-systemic elimination events and patient compliance. The latter factor penalizes, for example, the parenteral administration routes, which on one hand allow the drug to immediately reach the systemic circulation and rapidly promote the expected pharmacological effect but on the other hand are not very appreciated by the patient, particularly when the drug is used for the treatment of chronic diseases. When the drug is administered to elderly or pediatric patients or patients with swallowing difficulty, the administration routes involving oral cavity withouth requiring drug swallowing, i.e. perlingual, sublingual and buccal, are preferred. Recently, the use of oromucosal films, tablets and gels characterized by rapid disintegration of the pharmaceutical form within the oral cavity meets these requirements and has been widely applied. In particular, these formulations are useful when the active substance is capable of being directly absorbed by the oral mucosa characterized by a rich vascularization. Absorption through the oral mucosa allows speeding up the drug entry into the systemic circulation with respect to conventional gastrointestinal absorption and reduces pre-systemic elimination events, such as enzymatic or chemical degradation of the active substance in the gastrointestinal lumen and the first pass liver effect, responsible for reducing bioavailability and impairing drug effect. One of the problems encountered in the administration through the oral mucosa of slightly soluble or water insoluble active substances is their low solubility in the salivary environment. Several measures have been adopted to overcome this problem. In the literature it is well known, for example, that using cyclodextrins capable of complexing poorly soluble or insoluble active substances in an aqueous environment allows an easier administration thereof through the oral mucosa. Cyclodextrins (CDs) are produced from starch and comprise a family of cyclic oligosaccharides made up of 6, 7 or 8 monomers of D-(+)glucopyranose joined together by α,1-4 glucoside bond and closed in a ring. Cyclodextrins have three-dimensional hollow cone structure and, being based on the number of monomers: 6, 7, or 8, are referred as alpha (αCD), beta (βCD) or CD gamma (yCD). The three classes of CD differ in their ring size and therefore in their cavity. The hydroxyl groups are arranged on the outer edges, while only hydrogen atoms and oxygen bridges are present within the cavity. This causes the central cavity to have hydrophobic nature, while the external part characterized by the presence of hydroxyl groups has high hydrophilicity. Their particular structure allows to host hydrophobic molecules within the cavity making them soluble in an aqueous environment. The solubility of the CD has been further improved by chemical modifications in position 2,3 and 6 of the hydroxyl groups to give alkyl ethers or by introducing new functional groups. Among the chemically modified cyclodextrins there is the hydroxypropyl-β-cyclodextrin (HPβCD) that, given the high solubility with respect to the native βCD, has been largely used in the pharmaceutical field. Among the slightly soluble or water insoluble active substances that by complexation with cyclodextrins are able to be solubilized in aqueous media, there are steroid hormones. For example, US Patent No 4,596,795 describes solid pharmaceutical forms for buccal administration containing inclusion complexes between steroid hormones and HPβCD, obtained by freeze-drying; the authors do not use sucralose and the complexation reaction is not performed in presence of this agent. EP Patent No 2605778 B1 describes aqueous solutions for the administration of testosterone through oral mucosa comprising testosterone and randomized methyl β cyclodextrin. Cyclodextrin complexation greatly increases water solubility of the drug, without obtaining an equally strong permeability increase through the buccal mucosa; therefore, part of the dissolved drug accumulates in the oral cavity wherefrom it can be dispersed in the gastro-intestinal tract through accidental swallowing by the patient. US Patent No. 9066858 B2 describes sublingual tablets containing progesterone complexed in cyclodextrin, associated with an effervescent pair (citric acid/sodium bicarbonate); the effervescent pair facilitates the disintegration of the tablet and thus further increases the drug dissolution into oral cavity, resulting in a modest increase of absorbed drug.

The above mentioned patents highlight the effort in identifying an appropriate orodispersible formulation which could allow a quick release of poorly water soluble active substance, such as steroids, resulting in immediate availability for absorption thereof by the oral mucosa. Nevertheless there is still felt the need for oromucosal preparations which maximize actual drug bioavailability and efficacy to a higher patient benefit.

Sucralose is an artificial sweetener. In the European Union is also known as E955. Sucralose is produced by selective chlorination of sucrose, i.e. by selective replacement of three sucrose hydroxyl groups with an equal number of chlorine atoms, thus producing 1,6-dichloro-1,6-dideoxy-β-D-fructo-furanosil-4-chloro-4-deoxy-β-D-galactopyranoside. Sucralose, in the anhydrous state, tends to discolor in response to high temperatures; this problem has been solved by the Patent No. EP0375122B1: it describes complexes of crystallized sucralose with minor amounts of β-cyclodextrin (around 5-20%), useful for stabilizing sucralose against thermal degradation; the patent does not address drug bioavailability difficulties and does not describe other effects of the modified sucralose, except the sweetener one.

### SUMMARY

The invention is defined by the claims. Any subject-matter falling outside the scope of the claims is provided for information purposes only. The present inventors have now identified a new and unexpected effect of sucralose as pharmaceutically permeation enhancer of active ingredients (APIs) through the oral mucosa; this effect become evident when API is complexed with HPβCD in the presence of sucralose. In such conditions sucralose acts as a strong API absorption "enhancer" through the oral mucosa. The increase of API absorption involves an increase of its bioavailability. Object of the invention is therefore a new association ("composite" as defined herein), having high bioavailability, consisting of hydroxypropyl-β-cyclodextrin (HPβCD), sucralose, an active pharmaceutical ingredient (API), optionally an aqueous vehicle, wherein said API is complexed in said HPβCD, said composite having a molar ratio HPβCD:sucralose between 1:0.05 and 1:0.8, said API being a steroid. The composite is obtainable through dissolution of its three components in an aqueous vehicle, using methods described later, and optional elimination of the aqueous vehicle by means of known techniques, in particular freeze-drying or spray-drying. The composite obtained in such a way can be used as an ingredient in the preparation of pharmaceutical compositions for oromucosal administration (oromucosal tablets, films, gels, etc.).

### DESCRIPTION OF FIGURES

Figure 1:
   Comparative profiles, obtained as described in Example 3, showing the cumulative amount of testosterone permeated through buccal epithelial cells of a ternary composite of Testosterone/HPβCD and sucralose prepared according to the invention (Example 1) vs. a binary complex of HPβCD and Testosterone (Example 2), vs. a physical mixture of Testosterone and sucralose in the absence of HPβCD.
Figure 2:
   Comparative profiles, obtained as described in Example 3, showing the Testosterone absorption rate through buccal epithelial cells of a ternary composite Testosterone/HPβCD and sucralose prepared according to the invention (Example 1) vs. a binary complex comprising the sole complex of HPβCD and Testosterone (Example 2).

### DETAILED DESCRIPTION OF THE INVENTION

The term "composite" refers to a product consisting in the following substances: sucralose, hydroxypropyl β-cyclodextrin, API as above defined, and optionally an aqueous medium, wherein the API is complexed within hydroxypropyl β-cyclodextrin, in accordance with the invention. Preferably, the composite is a solid composite (being in its final solid form, not including the acqueous vehicle or any other liquid).

The term "in complexed form" means that API is present within the molecular structure of HPβCD, as commonly achievable by forming known complexes between drugs and HPβCD.

The term "oromucosal administration" refers to a treatment method wherein the administration and the subsequent drug absorption occur within the buccal cavity: therein the drug is released from the pharmaceutical form and is substantially absorbed into the bloodstream through the mucosa belonging to the oral cavity (mouth, tongue, sublingual area, gums, throat, etc.). Such administration is typically characterized by the use of pharmaceutical forms such as sublingual tablets, films, adhesives gels, troches, etc., which may be maintained in the oral cavity for a sufficient time enabling the progressive release of the drug into it. The oromucosal administration is therefore distinct from the oral administration in which the drug is swallowed/drunk and absorbed into the circulation at the stomach and/or intestine level. The oromucosal administration is further differentiated from the oral one in the fact that the drug absorbed into the circulation through the buccal mucosa reaches the tissues more quickly (due to high vascularity of the buccal mucosa) and directly, that is, avoiding the first pass metabolism through the liver (*first pass metabolism,* which is instead typical of the oral administration).

The term "slightly soluble" in water, or "water-insoluble" is herein defined according to the solubility classification reported by *European Pharmacopoeia* eighth edition effective from January 1, 2014. In particular, object of the present patent are APIs that require at least 100 mL of water to dissolve 1 g of API, at a temperature of 20°C. The active substances formulated according to the invention have improved stability, solubility and palatability in the oral cavity.

The hydroxypropyl β-cyclodextrin (HPβCD) used in the invention has preferably a degree of molar substitution (MS) within the range: 0.4-1.5. Molar degree of substitution means the number of hydroxypropoxyl groups present per D-(+) glucopyranose unit.

The present invention is advantageously used when the above defined API is slightly soluble in water or water-insoluble, conditions in which the increased bioavailability following buccal administration is particularly appreciated.

As above defined, according to the present invention, the API is a steroid. The steroid can be selected from, but not limited to, the following classes: steroidal anti-inflammatory drugs, steroid hormones and mixtures thereof. Among the steroid hormones, preferred are testosterone, progesterone, and derivatives thereof; one of their preferred formulation is as oromucosal tablets.

The composites of the invention are characterized in that: (a) the molar ratio hydroxypropyl-P-cyclodextrin:sucralose is between 1:0.05 and 1:0.8. Optionally, said composites may be further characterized in that: (b) the molar ratio API:hydroxypropyl-β-cyclodextrin is between 1:1 and 1:4 and/or (c) the molar ratio API:sucralose is between 1:0.05 and 1:2.

More preferred embodiments are those in which at least one among the optional conditions (b) and (c) is fulfilled; particularly preferred embodiments are those in which both the optional conditions (b) and (c) are fulfilled.

The condition (a) characterizes the composite of the present invention: in these composites, the amount of HPβCD is considerably larger compared to sucralose: this product is functionally definable as "HPβCD modified with sucralose", and not the other way around.

The optional conditions (b) and (c) define the relationship between API and the other composite components. They are intended as calculated on the basis of the whole API present into composite.

When the aqueous vehicle is present in the composite, it is water or a mixture of water with a water-miscible solvent, e.g. alcohol, glycol, etc.

In the absence of the aqueous vehicle, the composite of the invention is in solid form, being a preferred embodiment of the invention: it is typically not crystalline and may be further characterized as an amorphous product. The absence of crystalline structure and the amorphous nature of the product can be detected by microscope observation and can be analytically confirmed by known techniques, in particular, X-ray diffraction spectroscopy, infrared spectroscopy, etc. A preferred amorphous form is the one that is obtainable from the corresponding aqueous solutions by lyophilization or spray-drying.

A further object of the invention is a process for the preparation of the composite described above. It is characterized by comprising a step of complexation of the API as above defined, in the presence of sucralose. In a preferred mode, it includes the following steps:
(a) dissolving HPβCD in an aqueous vehicle
(b) dissolving sucralose in the solution obtained in (a).
(c) adding said API in the solution obtained in (b).
(d) optionally, removing the aqueous vehicle.

The steps (a) and (b) are reversible, obtaining the following process variant:
(a) dissolving sucralose in an aqueous vehicle
(b) adding HPβCD to the solution obtained in (a).
(c) adding said API to the solution obtained in (b).
(d) optionally, removing the aqueous vehicle.

In the above process, the aqueous vehicle may be water or a mixture thereof with another water-miscible solvent, e.g. alcohol, glycol, etc. In steps (b), the term "adding" is widely understood in the sense that the substance mentioned therein can be added in solid form and dissolved in the solution obtained in (a), or it can be separately dissolved in an aliquot of aqueous vehicle and added as such to the solution obtained in (a); in any case the step (b) ends with obtaining a solution.

The molar ratio between the amounts of HPβCD and sucralose used in steps (a) and (b) can vary between 1:0.05 and 1:0.8.

The step (c), it always subsequent to the steps (a) and (b), thus ensuring that the complexation of said API with HPβCD always takes place in the presence of sucralose. In step (c) said API is added in a molar ratio with HPβCD such as to be effectively complexed in it: for this purpose, a preferred range of API:HPβCD molar ratio is between 1:1 and 1:4. The molar ratio of API:sucralose varies preferably between 1:0.05 and 1:2.

In the optional step (d) any technique suitable to eliminate an aqueous solvent, e.g. evaporation, distillation, freeze-drying, spray-drying, etc., can be used; freeze-drying and spray-drying are preferred.

A further object of the invention is a pharmaceutical composition comprising the composite of the invention described above. The composition can be, in particular, suitable for oromucosal administration; in particular, it can be in the form of powder, granulate, tablet, minitablet, pill, gel, or film.

Such compositions can be obtained by per se known techniques, e.g. the tablets can be prepared by: i) sieving (directly or previously processed) the components; ii) gradual mixing of powders; iii) direct compression. Depending on the particular pharmaceutical form, the preparation process may involve various techniques such as: fluid-bed granulation (top/bottom spray, rotogranulator), high share mixer granulation, dissolver.

The above compositions may optionally contain, in addition to the composite of the invention, additives in function of the type of formulation. Among them flavours, sweeteners, carriers, stabilizers, plasticizers, preservatives, surfactants, emulsifiers, film-forming agents, lubricants, dyes, etc. can be mentioned. The formulations of the present invention are prepared according to procedures known in the art.

The present invention includes a preparation process of a oromucosal pharmaceutical composition as described hereabove, characterized by mixing said composite with suitable excipients and/or pharmaceutical carriers.

The invention also comprises the composite and the compositions as here above described for use in the administration of active substances through the oral mucosa. More specifically, the invention includes the use in the medical field of said composite and compositions, i.e. for the use in a method of treatment and/or prevention of diseases responsive to the specifically used active substances. For example, when the steroidal API in accordance with the invention is a steroid hormone, e.g. testosterone, the composite or composition is intended for the use in a method of treatment of the pathologies characterized by a reduced production of said hormone.

The present composites, once administered into the buccal cavity, in contact with saliva, lead to high and unexpected transmucosal absorption of the API as above defined. From experimental tests reported herein, the increased permeation is clearly due to the presence of sucralose, and can be observed exclusively when said API is complexed; without being bound by theory, the effect seems to be primarily resident in an interaction between cyclodextrin and sucralose. Furthermore, in the experimental tests conducted by the Applicant, the enhancement effect was seen independently from the dissolution time of the solid form: this shows that the present invention tends to reduce the residence time of the drug dissolved in the oral cavity, rather than increasing the dissolution rate of the solid pharmaceutical form. Thus a targeted effect is achieved thereby the API dissolved in the oral cavity is more quickly absorbed by the oral mucosa: this avoids the accumulation of dissolved API in the oral cavity, which would be easily washed away by saliva and/or accidental swallowing reducing the amount of bioavailable API through the buccal route.

The invention is described hereinafter by means of the following examples.

### EXPERIMENTALS

By way of example, some of the possible applications in oromucosal preparations benefiting from this discovery will be described. In particular, formulations as oromucosal tablets for buccal administration comprising testosterone will be described. The pharmaceutical forms thus produced more are easily used by the patient than other formulations, particularly in cases of chronic treatment.

### EXAMPLE 1: Preparation of the ternary composite: Testosterone/HPβCD/sucralose (molar ratio: 1.0 : 2.0 : 0.2)

278.95 g of deionized water are poured in a dissolver at room temperature, 279.62 g of hydroxypropylbetacyclodextrine are added and solubilized by stirring at 500 rpm until complete dissolution, so that the solution appears clear and free of visible residues.

6.821g of Sucralose are added to the solution and mixed for 5 minutes at 500 rpm at room temperature until complete dissolution so that the solution appears clear and free of visible residues. 26.4 g of Testosterone are added to the solution under continuous stirring until complete dissolution (the solution appears clear and free of visible residues). The obtained solution is diluted with the remaining aliquot of water equal to 172.43 g. The mixture is stirred for 10 - 15 minutes at 500 rpm at room temperature in order to homogenize the solution.

Spray-drying procedure is performed in order to obtain the final powder (spray dry mixture).

### EXAMPLE 2: Preparation of the binary complex HPβCD/ Testosterone (molar ratio: 2.0 : 1.0)

278.95 g of deionized water are poured in a dissolver at room temperature, 279.62 g of hydroxypropylbetacyclodextrin are added and solubilized by stirring for 5 minutes at 500 rpm until complete dissolution, so that the solution appears clear and free of visible residues.

26.4 g of Testosterone are added to the solution under continuous stirring until complete dissolution (the solution appears clear and free of visible residues. The obtained solution is diluted with the remaining aliquot of water equal to 172.43 g. The mixture is stirred for 10 - 15 minutes at 500 rpm at room temperature in order to homogenize the solution. Spray-drying procedure is performed in order to obtain the final powder (spray dry mixture).

### EXAMPLE 3: Permeation study through epithelial cells

The absorption enhancement mechanism was confirmed by tests on in-vitro grown buccal epithelium using simulated saliva at pH 6.8 (M.R.C. Marques et al." Simulated Biological fluids with Possible application in dissolution testing" ; Dissolution Technologies August 2011).

### Simulated saliva at pH 6.8

8.00 g of Sodium Chloride, 0.19 g of monobasic potassium phosphate and 2.38 g of dibasic sodium phosphate are dissolved in 1 L of H₂0 under magnetic stirring, after solution acidification to pH 6.8 with 85% phosphoric acid.

### Sample solution 1: Ternary composite Testosterone/HPβCD and spray dried sucralose (molar ratio: 1.0: 2.0 : 0.2)

300 mg of ternary composite of Testosterone/HPβCD and spray dried sucralose are dissolved in 50 mL of simulated saliva at pH 6.8 under slow magnetic stirring. The final concentration of testosterone in the solution is equal to 0.516 mg/mL.

### Sample solution 2: Binary complex Testosterone/spray dried HPβCD (molar ratio: 1.0 : 2.0)

300 mg of binary complex of Testosterone/spray dried HPβCD are dissolved in 50 mL of simulated saliva at pH 6.8 under slow magnetic stirring. The final concentration of testosterone present in the solution is equal to 0.516 mg/mL.

### Sample mixture 3: physical mixture of Testosterone and sucralose in the absence of HPβCD (molar ratio: 1.0 : 0.2)

25.00 mg Testosterone are dispersed under magnetic stirring in a solution of 6.60 mg of Sucralose in 50 mL of simulated saliva at pH 6.8. The final concentration of testosterone present in the solution is equal to 0.500 mg/mL.

### Test on in-vitro grown buccal epithelium.

Prior to their use the buccal epithelium membranes have been removed from maintenance gel and preincubated 1 hour at 37°C in the presence of simulated saliva at pH 6.8. At the end of equilibration period the membranes were transferred into incubation wells for the absorption kinetics testing. In each receiving well, a 300 µL aliquot of simulated saliva at pH 6.8 was added, while at the top of the epithelium (donor well) a 300 µL aliquot of a test sample solution was added. The kinetics was monitored for 0, 1, 2, 3, 4, 5 and 6 hours at 37°C.

At each sampling time, the membranes were moved to the next well, where 300 µL of simulated saliva were previously added. After the membrane movement, a sample aliquot of 50 µL was collected from the receiving well, diluted to 1000 µL with simulated saliva at pH 6.8 and sent for chromatographic analysis using a validated HPLC-UV method.

5 buccal membranes were employed for evaluating the absorption kinetics of **sample solution 1;** 5 membranes for **sample solution 2,** one membrane for **sample mixture 3** and one membrane for the solution of **simulated saliva at pH 6.8** as blank, respectively. The evaluation test of the absorption kinetics was performed simultaneously for all the formulations under study.

The HPLC-UV chromatographic analysis was performed in isocratic flow at 25°C using a YMC Pack Pro C18 column 3µm 4.0 x 150 mm with Acetonitrile:Water in 42 : 58 ratio as mobile phase. Operating wavelength was 244 nm, flow 1 mL/min and injection volume 20µL.

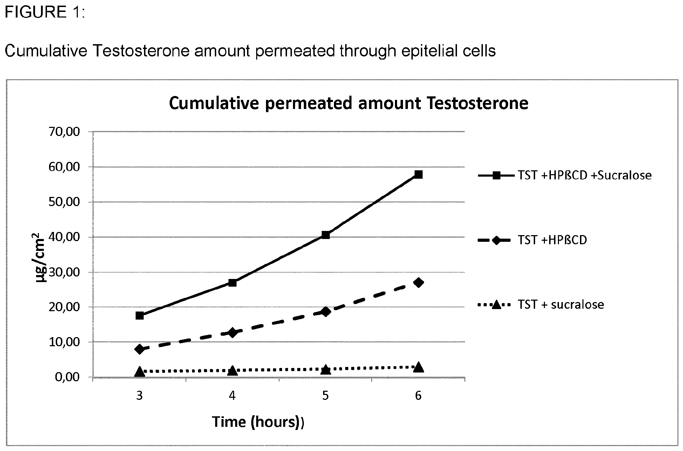

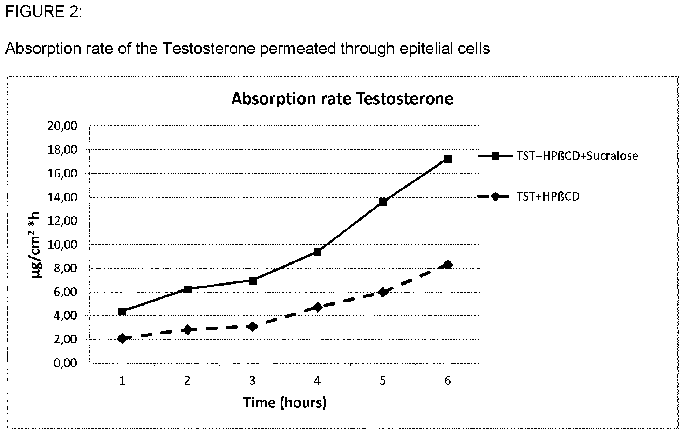

The absorption enhancement mechanism of the present invention was confirmed by tests on in-vitro grown buccal epithelium, see fig. 1 and 2. In particular figure 1 shows the comparative profiles of cumulative testosterone amount permeated through epithelial cells of a ternary composite Testosterone/HPβCD and sucralose prepared according to the invention (Example 1) and completely solubilized in simulated saliva vs. a binary complex completely solubilized in simulated saliva, comprising the HPβCD and Testosterone complex alone (Example 2), vs. the partially dissolved physical mixture of testosterone and sucralose in simulated saliva.

The comparative profiles shown in Figures 1 and 2 highlight the enhancer effect of sucralose on absorption through the oral mucosa.

In particular, data shown in Figures 1 and 2 highlight a very clear increase of absorption/permeation rate when switching from a drug complex in HPβCD (known) to a drug composite complexed in HPβCD in the presence of sucralose (according to the present invention). This activity is totally different from the one known for sucralose (sweetener). Furthermore, the effects shown in figures 1 and 2 were obtained by complexes previously solubilized in an aqueous medium, thereby eliminating a variability of results in function of the dissolution rate in water of the complex from the solid state: thus data show a greater tendency of this composite to oromucosal permeation, which is not derived from a more rapid dissolution of the solid form. Furthermore, the testosterone permeation data in the presence of sucralose alone (i.e. in the absence of HPβCD), were extremely low: this shows that sucralose has not a general enhancer effect with respect to the free drug; it becomes instead evident when the drug is complexed in HPβCD: thus the interaction useful for permeation enhancement seems mainly directed towards the cyclodextrin; this supports the effectiveness of the invention also when using different APIs, as long as they are complexed in HPβCD.

### EXAMPLE 4: Oromucosal gel containing the ternary composite Testosterone (unit dosage 2 mg), Hydroxypropyl-β-cyclodextrin, Sucralose.

The gel is obtained from two distinct phases (phase A and B) which are appropriately mixed. The example reports the procedure for preparating a single-dose stick (or multidose with dispenser) of oromucosal gel.

All components are weighed in the amounts described in Table 1.

### Preparation procedure of phase A (Molar ratio: HPβCD:Testosterone: Sucralose= 1.9 : 1.0: 0.3)

2 kg of deionized water are loaded into a appropriately large dissolver equipped with electromechanical stirrer and 0.09 Kg of Sucralose are solubilized thereinto; after complete dissolution at a mixing speed between 700-1000 rpm at room temperature 2 kg of hydroxypropyl beta-cyclodextrin are added (HPβCD; degree of substitution 0.87) at a mixing speed between 700-1000 rpm. When dissolution of HPβCD is complete (estimated time around 60 minutes) and a clear and free of visible residues solution is obtained, 0.2 Kg Testosterone are added under stirring (1000 rpm); stirring is continued until complete solubilization (time required about 60 minutes). After complete dissolution the stirring is maintained (100 - 200 rpm).

### Preparation procedure of phase B

4.1 kg of deionized water are charged in a planetary dissolver and under vacuum and a temperature of 50°C ± 5°C is set. As the set temperature is reached the planetary is activated at a speed of 60 rpm and 0.4 Kg of Polivinilalcohol 5 cp are added. As complete dissolution of the component is reached, while maintaining a mixing speed of 60 rpm, 4.1 kg of deionized water are poured and, at constant speed and temperature, 0.63 kg of Hydroxypropyl methylcellulose 5 p are added under stirring.

After complete dissolution, under constant agitation, the following amounts are poured in order: 4.1 kg of deionized water, 0.03 kg of chitosan glutamate, 0.1 kg of potassium sorbate (0.34%) and 0.2 kg of sodium benzoate (0.67%). The pH of the mixture was monitored (pH 4 to 5) and corrected, as appropriate.

After complete dissolution the mass contained in the planetary is kept under stirring and heated to a temperature of 50°C ± 5°C.

### Joining procedure of phase A and phase B

Phase B is drawn into phase A and mixing is continued at a speed of 50 rpm. Maintaining a constant mixing speed, the preparation is completed by adding 0.35 kg of liquid flavor and 11.2 kg of deionized water. The process is stopped once it reaches a complete homogenization of the two phases.

The formulation of the oromucosal gel containing 2 mg Testosterone is shown in Table 1

**Table 1**

| **Components** | **mg/unit dose** | **Kg/batch** |
|---|---|---|
| Hydroxypropyl beta-cyclodextrin (HPβCD) | 20.0 | 2.0 |
| Testosterone | 2.0 | 0.2 |
| Polyvinyl alcohol 5 cp (PVA 5cp) | 4.0 | 0.4 |
| Hydroxypropyl methylcellulose 5 p (HPMC 5p) | 6.3 | 0.63 |
| Chitosan glutamate | 0.3 | 0.03 |
| Potassium sorbate (K sorbate) (0.34%) | 1.0 | 0.1 |
| Sodium benzoate (Na Benzoate) (0.67%) | 2.0 | 0.2 |
| Sucralose | 0.9 | 0.09 |
| Flavor | 3.5 | 0.35 |
| Deionised water | 255 | 25.5 |
| Total dry weight | 295.0 | 29.5 |

### Example 5: Oromucosal Granular

Described below is the preparation of a oromucosal granular obtained by fluid bed granulation containing the ternary composite of Testosterone (unit dosage 2 mg), Hydroxypropyl-β-cyclodextrin, Sucralose.

The obtained product is stored in sachets (Al/paper/PE).

All components are weighed in the amounts described in Table 2.

### Preparation of Granulating Solution (Molar ratio: HPβCD:Testosterone: Sucralose= 2.1 : 1.0 : 0.2)

2.128 kg of deionized water are poured in a dissolver at room temperature, 2.18 Kg of hydroxypropylbetacyclodextrin are added and solubilized by stirring for 30 minutes at 500 rpm until complete dissolution, so that the solution appears clear and free of visible residues.

0.052 Kg of Sucralose are added to the solution and mixed for 5 minutes at 500 rpm at room temperature until complete dissolution so that the solution appears clear.

0.2 kg of Testosterone are added to the solution under stirring. Stirring is continued for a maximum of 4 hours until complete dissolution (the solution appears clear and free of visible residues)

### Preparation of the granulated phase

With the aim of creating the basic granular, sieve the formulation excipients (7.184 Kg of Neosorb and 0.038 Kg of sweetener) through a 0.8 mm of net light grid and load the powders into the fluid-bed granulator drum sieve. Set the granulation conditions (process temperature and air flow speed) in order to best perform the process.

The obtained granular is flowing.

### Preparation of the external phase

Perform the preparation of the external phase by placing the following amounts of excipients (in particular: 0.3 kg of citric acid, 0.3 kg of sodium bicarbonate, 0.798 Kg of flavor) into a stainless steel container for mixing by previously sieving them on a 0.8 mm grid.

Mix for 10 minutes at a speed of 10 rpm.

### Preparation of the final mixture

The preparation of the final mixture comprises transferring the granulated phase in the container, where the external phase is present, and mixing the whole for 30 minutes at a mixing speed of 10 rpm.

The complete mixture is packed in suitable sealed bags.

The formulation of the oromucosal granular containing 2 mg Testosterone is shown in Table 2

**Table 2**

| **Components** | **mg/unit dose** | **Kg/batch** |
|---|---|---|
| **Granular solution** | | |
| Hydroxypropyl beta-cyclodextrin (HPβCD) | 21.8 | 2.18 |
| Testosterone | 2.0 | 0.2 |
| Sucralose | 0.52 | 0.052 |
| Ultra-filtered water | 21.28 | 2.128 |

| **Granulating phase** | | |
|---|---|---|
| Sorbitol (Neosorb 100) | 71.84 | 7.184 |
| Sweetener | 0.38 | 0.038 |

| **External phase** | | |
|---|---|---|
| Citric acid | 3.0 | 0.3 |
| Sodium bicarbonate | 3.0 | 0.3 |
| Flavor | 7.98 | 0.798 |
| **Total (dry weight)** | 110 | 11.0 |

### EXAMPLE 6:Oromucosal tablet

The case described hereinbelow relates to the preparation of a oromucosal tablet obtained from a granular made by fluid bed granulation; this pharmaceutical form contains the ternary composite of Testosterone (unit dosage 2 mg), Hydroxypropyl-β-cyclodextrin, Sucralose.

The product obtained is kept in a Al/Al peeling blister containing 4 tablets or in a sealed HDPE bottle with desiccant.

All components are weighed in the amounts described in Table 3.

### Preparation of the granulating solution (Molar ratio: HPβCD:Testosterone: Sucralose= 2.1 : 1.0 : 0.2)

2.128 kg of deionized water are poured in a dissolver at room temperature, 2.128 Kg of hydroxypropylbetacyclodextrin are added and solubilized by stirring for 30 minutes at 500 rpm until complete dissolution, so that the solution appears clear and free of visible residues.

Add 0.052 Kg of Sucralose to the solution and mix for 5 minutes at 500 rpm at room temperature until complete dissolution so that the solution appears clear.

Add 0.2 kg of Testosterone to the solution under stirring. Continue stirring for a maximum of 4 hours until complete dissolution (the solution appears clear and free of visible residues)

### Preparation of the granulated phase

With the aim of creating the basic granular, sieve the formulation excipients (7.184 Kg of Neosorb and 0.038 Kg of Sweetener) through of a 0.8 mm of net light grid and load the powders into the fluid-bed granulator drum sieve. Set the granulation conditions (process temperature and air flow speed) in order to best perform the process.

The obtained granular is flowing.

### Preparation of the external phase

Perform the preparation of the external phase by placing the following amounts of excipients (in particular: 0.3 kg of citric acid, 0.3 kg of sodium bicarbonate, 0.798 Kg of flavor) into a stainless steel container for mixing by previously sieving them on a 0.8 mm grid.

Mix for 10 minutes at a speed of 10 rpm.

### Preparation of the complete mixture

The preparation of the complete mixture comprises transferring the granulating phase in the container where the external phase is present. Then mix the whole for 20 minutes at a mixing speed of 10 rpm.

Add 0.2 kg of magnesium stearate (vegetable origin) to the mixture obtained above, after sieving on a 0.2 mm sieve mesh. Proceed to the final mixing for 10 minutes at the speed of 10 rpm.

### Compression procedure:

With the aid of a rotary tablet press equipped with common round convex punches in tempered stainless steel of diameter 6.35 mm and bending radius R = 6 and forced loading hopper. Press the powder in order to obtain round tablets weighing 112 mg and a hardness between 20 - 40 N.

The formulation of the oromucosal tablet containing 2 mg Testosterone is shown in Table 3

**Table 3**

| **Components** | **mg/unit dose** | **Kg/batch** |
|---|---|---|
| **Granulating solution** | | |
| Hydroxypropyl beta-cyclodextrin (HPβCD) | 21.28 | 2.128 |
| Testosterone | 2.0 | 0.2 |
| Sucralose | 0.52 | 0.052 |
| Ultra-filtered water | 21.28 | 2.128 |

| **Granulating phase** | | |
|---|---|---|
| Sorbitol (Neosorb 100) | 71.84 | 7.184 |
| Sweetener | 0.38 | 0.038 |

| **External phase** | | |
|---|---|---|
| Citric acid | 3.0 | 0.3 |
| Sodium bicarbonate | 3.0 | 0.3 |
| Flavor | 7.98 | 0.798 |
| Magnesium stearate (vegetable source) | 2.0 | 0.2 |
| **Total (dry weight)** | 112 | 11.2 |

### EXAMPLE 7: Chewable oromucosal tablet

The case described hereinbelow relates to the preparation of a chewable oromucosal tablet obtained from a granular made by fluid bed granulation; this pharmaceutical form contains the ternary composite of Testosterone (unit dosage 2 mg), Hydroxypropyl-β-cyclodextrin, Sucralose.

The product obtained is kept in a Al/Al peeling blister containing 4 tablets or in a sealed HDPE bottle with desiccant.

All components are weighed in the amounts described in Table 4.

### Preparation of the granulating solution (Molar ratio: HPβCD:Testosterone: Sucralose= 2.1 : 1.0 : 0.2)

2.128 kg of deionized water are poured in a dissolver at room temperature, 2.128 Kg of hydroxypropylbetacyclodextrin are added and solubilized by stirring for 30 minutes at 500 rpm until complete dissolution, so that the solution appears clear and free of visible residues.

Add 0.052 Kg of Sucralose to the solution and mix for 5 minutes at 500 rpm at room temperature until complete dissolution so that the solution appears clear.

Add 0.2 kg of Testosterone to the solution under stirring. Continue stirring for a maximum of 4 hours until complete dissolution thereof (the solution appears clear and free of visible residues)

### Preparation of the granulated phase

With the aim of creating the basic granular, sieve the formulation excipients (7.184 Kg of Neosorb and 0.038 Kg of sweetener, 0.851 kg of CEOLUS 1000 kg, 1.9868 kg of Rxcipients FM 1000, 0.665 Kg Sodium Bicarbonate, 1.516 kg of Kollidon Cl) through a 0.8 mm of net light grid and load the powders in the fluid-bed granulator drum sieve. Set the granulation conditions (process temperature and air flow speed) in order to best perform the process.

The obtained granular is flowing.

### Preparation of the external phase

Perform the preparation of the external phase by placing the following amounts of excipients into a stainless steel container for mixing by previously sieving on the 0.8 mm grid, in particular: 0.625 kg of citric acid, 0.3 kg of sodium bicarbonate, 0.798 Kg of flavor.

Mix for 10 minutes at a speed of 10 rpm.

### Preparation of the complete mixture

The preparation of the complete mixture comprises transferring the granulating phase in the container where the external phase is present. Then mix the whole for 20 minutes at a mixing speed of 10 rpm.

Add 0.133 kg of magnesium stearate to the mixture obtained above, after sieving on a 0.2 mm sieve mesh. Proceed to the final mixing for 10 minutes at the speed of 10 rpm.

### Compression procedure:

With the aid of a rotary tablet press equipped with common round convex punches in tempered stainless steel of diameter 6.35 mm and bending radius R = 6 and forced loading hopper. Press the powder in order to obtain round tablets weighing 125 mg and a hardness between 20 - 40 N.

The formulation of the oromucosal tablet containing 2 mg Testosterone is shown in Table 4

**Table 4**

| **Components** | **mg/dose** | **Kg/batch** |
|---|---|---|
| **Granular solution** | | |
| Hydroxypropyl beta-cyclodextrin (HPβCD) | 21.128 | 2.128 |
| Testosterone | 2.0 | 0.2 |
| Sucralose | 0.52 | 0.052 |
| Ultra-filtered water | 21.128 | 2.128 |

| **Granulating phase** | | |
|---|---|---|
| Sorbitol (Neosorb 100) | 35.26 | 3.526 |
| Sweetener | 0.38 | 0.038 |
| Microcrystalline cellulose (Ceolus Kg 1000) | 8.51 | 0.851 |
| Calcium silicate (Rxcipients FM 1000) | 19.68 | 1.968 |
| Sodium bicarbonate | 6.65 | 0.665 |
| Polyvinylpyrrolidone, Povidone (Kollidon Cl) | 15.16 | 1,516 |

| **External phase** | | |
|---|---|---|
| Citric acid | 6.25 | 0.625 |
| Flavor | 7.98 | 0.798 |
| Magnesium stearate (Pruv) | 1.33 | 0.133 |
| **Total (dry weight)** | 125 | 1.25 |

### EXAMPLE 8: Oromucosal Film

The case described hereinbelow relates to the preparation of an oromucosal film containing the composite of Testosterone, Hydroxypropyl-β-cyclodextrin, Sucralose

The preparation comprises the preparation of two distinct phases A and B. The final product is obtained by combination of the two. The example shows the procedure for the preparation of a batch of 278.8 mg oromucosal films (100000 units) with Testosterone as active substance (unit dosage 2 mg). The films obtained are individually packaged in aluminum/polyethylene bags.

All components are weighed in the amounts described in Table 5.

### Preparation procedure of phase A (Molar ratio: HPβCD:Testosterone: Sucralose = 1.9 : 1.0 : 1.0)

In an electromechanical dissolver of adequate capacity 2.0 kg of deionized water are loaded, 0.276 Kg of Sucralose are added and mixed at a speed between 700-1000 rpm at room temperature; after solubilisation, 2 kg of hydroxypropyl beta-cyclodextrin (HPβCD; average degree of substitution 0.87) are added without interrupting the agitation. At the end of the dissolution of HPβCD (time around 60 minutes) the whole Testosterone (0.2 Kg) is added, always under stirring (1000 rpm ") up to complete solubilization (time required about 60 minutes). After complete dissolution (the solution is clear and residue-free) the system is maintained under stirring (100 -200 rpm) and a residual vacuum of 800 mbar with residual pressure 200-1000 mbar is applied for 15 minutes in order to remove the air bubbles possibly previously incorporated.

### Preparation procedure of phase B

15 Kg of deionized water are charged in a planetary dissolver homogenizer and the temperature set to 40°C ± 5°C. As the set temperature has been reached the planetary is activated at a speed of 50 rpm and sweetener (0.138 Kg), Xylitol (1.8 Kg) and chitosan glutamate (0.15 Kg) are added. As the complete dissolution of the components is reached, while maintaining the mixing speed constant, 2 Kg of polyvinylalcohol 5 cp are solubilized (time required approximately 30 minutes).

After complete dissolution the mass contained in the planetary is kept under stirring and heated to a temperature of 50°C ± 5°C.

3.7 kg HPMC E5 Premium and 0.26 Kg Colloidal Silica are mixed in a mixer.

When the mass contained in the planetary reaches the desired temperature, a mixing speed of 100-200 rpm is set and the mixture of HPMC E5 Premium and silica is added to the system. After complete incorporation of the HPMC/silica mixture (time required about 60 minutes) a vacuum of 800 mbar is applied and the turbine is activated at speed of 2000 rpm for 5-10 minutes while maintaining the planetary at a speed of 50 rpm.

### Joining procedure of phase A and phase B

Mass A was drawn in mass B while keeping the planetary at a speed of 50 rpm and under vacuum. The system is subjected to mixing for 15 minutes. After this time, while keeping the planetary speed and the vacuum condition constant, the preparation is completed by adding the following excipients: Sorbitol 70% (1.2 Kg), anhydrous glycerin (2 Kg), Propylene glycol (1 Kg), Polysorbate 80 (0.2 Kg), liquid flavors and solid microcrystalline cellulose (0.5 Kg). As a homogeneous incorporation of all elements was reached (after about 15 minutes), the vacuum is broken and the mass unloaded into a spreading machine under a slight nitrogen flow. The preparation is spread on a mono-siliconized polyethylene film and dried in a hot air current (set point 40°C ± 5°C). The dried film supported on the film is wound on a 600 mm coil. The coil is mounted on a punching machine that produces individual disks with a diameter of 30 mm, thickness of about 0.45 ± 0.1 mm and mean weight of 267 ± 4 mg (5-12% of residual water), each containing 2 mg of Testosterone.

The formulation of the oromucosal film containing 2 mg Testosterone is shown in Table 5

**Table 5**

| **Components** | **mg/unit dose** | **Kg/batch** |
|---|---|---|
| Xylitol | 18 | 1.8 |
| Chitosan glutamate | 1.5 | 0.15 |
| Hydroxypropyl beta-cyclodextrin (HPβCD) | 20 | 2.0 |
| Testosterone | 2 | 0.2 |
| Sorbitol 70% | 12 | 1.2 |
| Microcrystalline cellulose | 5 | 0.5 |
| Anhydrous glycerin | 20 | 2 |
| Propylene glycol | 10 | 1 |
| Flavours | 3 | 0.3 |
| Sucralose | 2.76 | 0.276 |
| Polyvinylalcohol 5 cp | 20 | 2 |
| Hydroxypropyl methylcellulose (HPMC) E5 Premium | 37 | 3.7 |
| Sweetener | 1.38 | 0.138 |
| Colloidal silica | 2.6 | 0.26 |
| Polysorbate 80 | 2 | 0.2 |
| Deionised water | 255 | 25.5 |
| Total pre-drying | 412.24 | 41.224 |
| 100000 pieces (pcs) batch (dried weight) | 278.8 | 27.88 |

### EXAMPLE 9: Oromucosal film

The case described below concerns the preparation of an oromucosal film containing the composite of Testosterone, Hydroxypropyl-β-cyclodextrin, Sucralose

The preparation involves the preparation of two distinct phases A and B. The final product is obtained by combination of the two. The example shows the procedure for the preparation of a batch of 278.8 mg oromucosal films (100000 units) with Testosterone active principle (unit dosage 2 mg). The films obtained are individually packaged in aluminum/polyethylene bags.

All components are weighed in the amounts described in Table 6.

### Preparation procedure of phase A (Molar ratio: HPβCD:Testosterone: Sucralose = 1.9 : 1.0 : 1.0)

In an electromechanical dissolver of adequate capacity 2.0 kg of deionized water are loaded, 0.276 Kg of Sucralose are added and mixed at a speed between 700-1000 rpm at room temperature; after solubilization 2 kg of hydroxypropyl beta-cicodestrina (HPβCD; average degree of substitution 0.87) are added without interrupting the stirring. At the end of the dissolution of HPβCD (time around 60 minutes) the whole Testosterone (0.2 Kg) is added, always under stirring (1000 rpm) up to complete solubilization (time required about 60 minutes). After complete dissolution (the solution is clear and residue-free) the system is maintained under stirring (100 -200 rpm) and a residual vacuum of 800 mbar with residual pressure 200-1000 mbar is applied per 15 minutes in order to remove the air bubbles possibly previously incorporated.

### Preparation procedure of phase B

15 Kg of deionized water are charged in a planetary dissolver homogenizer and a temperature is set to 40°C ± 5°C. As the set temperature has been reached the planetary is activated at a speed of 50 rpm and sweetener (0.138 Kg), and chitosan glutamate (0.15 Kg) are added. As the complete dissolution of the components is reached, while maintaining the mixing speed constant, 2 Kg of Polyvinylalcohol 5 cp (time required approximately 30 minutes) are solubilized.

After complete dissolution the mass contained in the planetary is kept under stirring and heated to a temperature of 50°C ± 5°C.

3.7 kg HPMC E5 Premium and 0.26 Kg Colloidal Silica are mixed in a mixer.

When the mass contained in the planetary reaches the desired temperature, a mixing speed of 100-200 rpm is set and the mixture of HPMC E5 Premium and silica is added to the system. After complete incorporation of the HPMC/silica mixture (time required about 60 minutes) a vacuum of 800 mbar is applied and the turbine activated at a speed of 2000 rpm for 5-10 minutes while maintaining the planetary at a speed of 50 rpm.

### Joining procedure of phase A and phase B

Mass A was drawn in mass B while keeping the planetary at a speed of 50 rpm and under vacuum. The system is subjected to mixing for 15 minutes. After this time, while keeping the planetary speed and the vacuum condition constant, the preparation is completed by adding the following excipients: Sorbitol 70% (1.2 Kg), anhydrous glycerin (2.5 Kg), Propylene glycol (1.5 Kg), Polysorbate 80 (0.2 Kg), liquid flavors and solid microcrystalline cellulose (1.0 kg). As a homogeneous incorporation of all elements is reached (after about 15 minutes), the vacuum is broken and the mass unloaded into a spreading machine under a slight nitrogen flow. The preparation is spread on a mono-siliconized polyethylene film, and dried in a hot air current (set point 40°C ± 5°C). The dried film supported on the film is wound on a 600 mm coil. The coil is mounted on a punching machine that produces individual disks with a diameter of 30 mm, thickness of about 0.45 ± 0.1 mm and mean weight of 267 ± 4 mg (5-12% of residual water), each containing 2 mg of Testosterone.

The formulation of the oromucosal film containing 2 mg Testosterone is shown in Table 6

**Table 6**

| **Components** | **mg/dose** | **Kg/batch** |
|---|---|---|
| Chitosan glutamate | 1.5 | 0.15 |
| Hydroxypropyl beta-cyclodextrin (HPβCD) | 20 | 2.0 |
| Testosterone | 2 | 0.2 |
| Sorbitol 70% | 12 | 1.2 |
| Microcrystalline cellulose | 10 | 1 |
| Anhydrous glycerin | 25 | 2.5 |
| Propylene glycol | 15 | 1.5 |
| Flavours | 3 | 0.3 |
| Sucralose | 2.76 | 0.276 |
| Polyvinylalcohol 5 cp | 20 | 2 |
| Hydroxypropyl methylcellulose (HPMC) E5 Premium | 40 | 4 |
| Sweetener | 1.38 | 0.138 |
| Colloidal silica | 2.6 | 0.26 |
| Polysorbate 80 | 2 | 0.2 |
| Deionised water | 255 | 25.5 |
| Total pre-drying | 412.24 | 41.224 |
| 100000 pieces (pcs) batch (dried weight) | 278.8 | 27.88 |

### EXAMPLE 10: Oromucosal tablet

The case described hereinbelow relates to the preparation of a oromucosal tablet obtained from a spray-dried mixture; this pharmaceutical form contains the composite of Testosterone (unit dosage 1 mg of Testosterone), Hydroxypropyl-p-cyclodextrin, Sucralose.

The product obtained is kept in an Al/Al peeling blister containing 4 tablets or in a sealed HDPE bottle with desiccant.

All components are weighed in the amounts described in Table 7.

Here below the preparation of a 1650 g batch of 26400 oromucosal tablets of 1 mg of Testosterone is described.

### Preparation of the spray-dried powder mixture (Molar ratio: HPβCD: Testosterone: Sucralose = 2.0 : 1.0: 0.2)

278.95 g of deionized water are poured in a dissolver at room temperature, the whole hydroxypropylbetacyclodextrin is added and solubilized by stirring for 5 minutes at 500 rpm until complete dissolution, when the solution appears clear and free of visible residues.

Add 6.821g of Sucralose to solution and mix for 5 minutes at 500 rpm at room temperature until complete dissolution so that the solution appears clear and free of visible residues. Add 26.4 g of Testosterone to the solution under continuous stirring. Continue stirring until complete dissolution (the solution appears clear and free of visible residues). Dilute the obtained solution with the remaining aliquot of water equal to 172.43 g. Stir the mixture for 10 - 15 minutes at 500 rpm at room temperature in order to homogenize the solution.

Performe the spray-drying procedure in order to obtain the final powder (spray-dried mixture).

### Preparation of the final mixture

Sieve the obtained spray dried mixture and other formulation excipients (200.2 g of crospovidone, 105.6 g of Flavor, 82.5 g of citric acid, 88 g of sodium bicarbonate, 259.6 g of Calcium silicate, 466.334 g of sorbitol and 112.2 g of microcrystalline cellulose) on 1 mm net light grid.

Separately sieve the remaining aliquote of sweetener (5,125 g) on 0.5 mm grid.

Perform the preparation of the semifinished product into a stainless steel container by mixing the powders for 20 minutes at a speed of 10 rpm.

Sieve the lubricant (Sodium Stearyl Fumarate 17.6 g) on a 0.2 mm grid and add it to the mixture; mix for 5 minutes at a speed of 10 rpm.

### Compression procedure:

With the aid of a rotary tablet press equipped with common round convex punches in tempered stainless steel of diameter 5.35 mm and bending radius R = 6 and forced loading hopper. Press the powder in order to obtain round tablets weighing 62.5 mg and a hardness between 20 - 40 N.

The formulation of the oromucosal tablet containing 1 mg Testosterone is shown in Table 7

**Tabella7:**

| **Components** | **mg/unit dose** | **g/batch** |
|---|---|---|
| Water | * | 451.387 |
| Testosterone (spray dry) | 1.00 | 26.400 |
| Sorbitol | 17.63 | 466.334 |
| Sucralose | 0.258 | 6.821 |
| HP Beta Cyclodextrin (spray dry) | 10.64 | 279.620 |
| RxCipients FM 1000 (Calcium silicate) | 9.84 | 259.600 |
| Crospovidone | 7.58 | 200.200 |
| Microcrystalline cellulose | 4.255 | 112.200 |
| Sodium bicarbonate | 3.325 | 88.000 |
| Citric acid | 3.125 | 82.500 |
| Flavor | 4.00 | 105.6 |
| Sodium stearyl fumarate | 0.665 | 17.600 |
| Sweetener | 0.194 | 5.125 |
| Total | 62.50 | 1650.000 (dry weight) |

| | | |
|---|---|---|
| *Water evaporated during spray dry process | | |

### EXAMPLE 11: Oromucosal tablet

The case described hereinbelow concerns the preparation of an oromucosal tablet obtained from a spray-dried mixture; this pharmaceutical form contains the composite of Testosterone (unit dosage 12 mg of Testosterone), Hydroxypropyl-β-cyclodextrin, Sucralose.

The product obtained is kept in an Al/Al peeling blister containing 4 tablets or in a sealed HDPE bottle with desiccant.

All components are weighed in the amounts described in Table 8.

Below is described the preparation of a 1650 g batch of 2200 oromucosal tablets of 12 mg of testosterone.

### Preparation of the spray-dried powder mixture (Molar ratio: HPβCD: Testosterone: Sucralose = 2.0 : 1.0: 0.2)

278.95 g of deionized water are poured in a dissolver at room temperature, the whole hydroxypropylbetacyclodextrin is added and solubilized by stirring for 5 minutes at 500 rpm until complete dissolution, when the solution appears clear and free of visible residues.

Add 6.821g of Sucralose to the solution and mix for 5 minutes at 500 rpm at room temperature until complete dissolution so that the solution appears clear and free of visible residues. Add 26.4 g of Testosterone to the solution under continuous stirring. Continue stirring until complete dissolution (the solution appears clear and free of visible residues). Dilute the obtained solution with the remaining aliquot of water equal to 172.43 g. Stir the mixture for 10 - 15 minutes at 500 rpm at room temperature in order to homogenize the solution.

Performe the spray-drying procedure in order to obtain the final powder (spray dried mixture).

### Preparation of the final mixture

Sieve the obtained spray-dried mixture and other formulation excipients (200.2 g of crospovidone, 105.6 g of Flavor, 82.5 g of citric acid, 88 g of sodium bicarbonate, 259.6 g of Calcium silicate, 466.334 g of sorbitol and 112.2 g of microcrystalline cellulose) on 1 mm net light grid.

Separately sieve the remaining aliquote of sweetener (5,125 g) on 0.5 mm grid.

Perform the preparation of the semifinished product into a stainless steel container by mixing the powders for 20 minutes at a speed of 10 rpm.

Sieve the lubricant (Sodium Stearyl Fumarate 17.6 g) on a 0.2 mm grid and add it to the mixture; mix for 5 minutes at a speed of 10 rpm.

### Compression procedure:

With the aid of a rotary tablet press equipped with common round convex punches in tempered stainless steel of diameter 16 mm and forced loading hopper. Press the powder in order to obtain round tablets weighing 750 mg and a hardness between 20 - 40 N.

The formulation of the oromucosal tablet containing 12 mg Testosterone is shown in Table 8

**Table 8**

| **Components** | **mg/unit dose** | **g/batch** |
|---|---|---|
| Water | * | 451.387 |
| Testosterone (spray dry) | 12.00 | 26.400 |
| Sorbitol | 211.97 | 466.334 |
| Sucralose | 3.10 | 6.821 |
| HP Beta Cyclodextrin (spray dry) | 127.10 | 279.620 |
| RxCipients FM 1000 (Calcium silicate) | 118.00 | 259.600 |
| Crospovidone | 91.00 | 200.200 |
| Microcrystalline cellulose | 51.00 | 112.200 |
| Sodium bicarbonate | 40.00 | 88.000 |
| Citric acid | 37.50 | 82.500 |
| Flavor | 48.00 | 105.60 |
| Sodium stearyl fumarate | 8.00 | 17.600 |
| Sweetener | 2.33 | 5.125 |
| Total | 750.00 | 1650.000 (dry weight) |

| | | |
|---|---|---|
| * Water evaporated during spray dry process | | |

## Claims

1. Composite consisting of: hydroxypropyl-β-cyclodextrin (HPβCD), sucralose, an active pharmaceutical ingredient (API), optionally an aqueous vehicle, wherein said API is complexed in said HPβCD, said composite having a molar ratio HPβCD:sucralose between 1:0.05 and 1:0.8, said API being a steroid.

2. Composite according to claim 1, having a molar ratio API:HPβCD between 1:1 and 1:4

3. Composite according to claims 1-2, having a molar ratio API:sucralose between 1:0.05 and 1:2.

4. Composite according to claim 1-3, wherein the steroid is selected from testosterone, progesterone and their derivatives.

5. Process for preparing the composite of claims 1-4, comprising complexing said API in HPβCD, in the presence of sucralose.

6. Process according to claim 5, comprising the following steps:
a. dissolving HPβCD (or sucralose) in an aqueous vehicle.
b. dissolving sucralose (or HPβCD) in the solution obtained in step a.
c. adding said API in the solution obtained in step b.
d. optionally, removing the aqueous vehicle.

7. Process according to claims 5-6, wherein in step d. the aqueous vehicle is removed by freeze-drying or spray-drying.

8. Process according to claims 5-7, wherein the product obtained in step (d) is an amorphous solid.

9. Composite obtained by the process of claims 5-8.

10. A pharmaceutical composition comprising the composite described in any one of claims 1-4, 9.

11. Pharmaceutical composition according to claim 10, suitable for oromucosal administration.

12. Pharmaceutical composition according to claims 10-11, chosen from: powder, granulate, tablet, minitablet, pill, gel, film.

13. Composite or composition as described in claims 1-4, 9-12 for use in a method of treatment **characterized by** oromucosal administration.

14. Composite or composition for the use according to claim 13, wherein said API is a steroid hormone and said treatment is directed to a disease **characterized by** a reduced production of said hormone.

15. Composite or composition for the use according to claim 14, wherein said API is selected from testosterone or progesterone.

## Patentansprüche

1. Zusammensetzung bestehend aus: Hydroxypropyl-β-Cyclodextrin (HPβCD), Sucralose, einem aktiven pharmazeutischen Wirkstoff (API), optional einem wässrigen Träger, wobei der API in dem HPβCD komplexiert ist, wobei die Zusammensetzung ein molares Verhältnis von HPßCD:Sucralose zwischen 1:0.05 und 1:0.8 aufweist und wobei der API ein Steroid ist.

2. Zusammensetzung nach Anspruch 1, aufweisend ein molares Verhältnis von API:HPβCD zwischen 1:1 und 1:4.

3. Zusammensetzung nach einem der Ansprüche 1-2, aufweisend ein molares Verhältnis von API:Sucralose zwischen 1:0.05 und 1:2.

4. Zusammensetzung nach einem der Ansprüche 1-3, wobei das Steroid ausgewählt ist aus Testosteron, Progesteron und deren Derivaten.

5. Verfahren zum Herstellen der Zusammensetzung nach einem der Ansprüche 1-4, umfassend ein Komplexieren des API in HPβCD, in der Gegenwart von Sucralose.

6. Verfahren nach Anspruch 5, umfassend die folgenden Schritte:
a. Auflösen von HPβCD (oder Sucralose) in einem wässrigen Träger.
b. Auflösen von Sucralose (oder HPβCD) in der in Schritt a. erhaltenen Lösung.
c. Hinzufügen des API zu der in Schritt b. erhaltenen Lösung.
d. optional, Entfernen des wässrigen Trägers.

7. Verfahren nach einem der Ansprüche 5-6, wobei der wässrige Träger in Schritt d. durch Gefriertrockenen oder Sprühtrocknen entfernt wird.

8. Verfahren nach einem der Ansprüche 5-7, wobei das in Schritt d. erhaltene Produkt ein amorpher Feststoff ist.

9. Zusammensetzung erhalten durch das Verfahren nach einem der Ansprüche 5-8.

10. Eine pharmazeutische Zusammensetzung, umfassend die Zusammensetzung beschrieben in einem der Ansprüche 1-4, 9.

11. Pharmazeutische Zusammensetzung nach Anspruch 10, geeignet zur oromukosalen Verabreichung.

12. Pharmazeutische Zusammensetzung nach einem der Ansprüche 10-11, ausgewählt aus: Pulver, Granulat, Tablette, Minitablette, Pille, Gel, Film.

13. Zusammensetzung oder Zusammensetzung wie in einem der Ansprüche 1-4, 9-12 beschrieben, zur Verwendung in einem Behandlungsverfahren, **gekennzeichnet durch** oromukosale Verabreichung.

14. Zusammensetzung oder Zusammensetzung für die Verwendung nach Anspruch 13, wobei der API ein Steroidhormon ist und die Behandlung auf eine Erkrankung, die durch eine verringerte Produktion des Hormons gekennzeichnet ist, gerichtet ist.

15. Zusammensetzung oder Zusammensetzung für die Verwendung nach Anspruch 14, wobei der API ausgewählt ist aus Testosteron oder Progesteron.

## Revendications

1. Composite constitué de : hydroxypropyl-β-cyclodextrine (HPβCD), sucralose, un agent actif pharmaceutique (API), optionnellement un véhicule aqueux, dans lequel ledit API est complexé dans ladite HPβCD, ledit composite ayant un ratio molaire HPβCD:sucralose compris entre 1:0.05 et 1:0.8, ledit API étant un stéroïde.

2. Composite selon la revendication 1, ayant un ratio molaire API : HPβCD compris entre 1:1 et 1:4.

3. Composite selon les revendications 1-2, ayant un ratio molaire API:sucralose compris entre 1:0.05 et 1:2.

4. Composite selon les revendications 1-3, dans lequel le stéroïde est choisi parmi la testostérone, la progestérone et leurs dérivés.

5. Procédé pour la préparation du composite selon les revendications 1-4, comprenant la complexation dudit API dans la HPβCD, en présence de sucralose.

6. Procédé selon la revendication 5, comprenant les étapes suivantes :
a. dissolution de la HPβCD (ou du sucralose) dans un véhicule aqueux ;
b. dissolution du sucralose (ou de la HPβCD) dans la solution obtenue à l'étape a ;
c. ajout dudit API dans la solution obtenue à l'étape b ;
d. optionnellement, retrait du véhicule aqueux.

7. Procédé selon les revendications 5-6, dans lequel à l'étape (d) le véhicule aqueux est retiré par lyophilisation ou par séchage par pulvérisation.

8. Procédé selon les revendications 5-7, dans lequel le produit obtenu à l'étape (d) est un solide amorphe.

9. Composite obtenu par le procédé selon les revendications 5-8.

10. Une composition pharmaceutique comprenant le composite tel que décrit à l'une quelconque des revendications 1-4, 9.

11. Composition pharmaceutique selon la revendication 10, adaptée pour une administration oromucosale.

12. Composition pharmaceutique selon les revendications 10-11, choisie parmi : une poudre, des granulés, un comprimé, un mini-comprimé, une pilule, un gel, un film.

13. Composite ou composition tel(le) que décrit(e) aux revendications 1-4, 9-12 pour une utilisation dans une méthode de traitement **caractérisée par** une administration oromucosale.

14. Composite ou composition pour une utilisation selon la revendication 13, dans lequel ou laquelle ledit API est une hormone stéroïde et ledit traitement est dirigé vers une maladie **caractérisée par** une production réduite de ladite hormone.

15. Composite ou composition pour une utilisation selon la revendication 14, dans lequel ou laquelle ledit API est choisi parmi la testostérone ou la progestérone.
